# EUROPEAN PATENT APPLICATION

(11) **EP 4 246 127 A1**
(43) Date of publication of application: **20.09.2023**
(21) Application number: 23161779.6
(22) Date of filing: 14.03.2023
(51) Int. Cl.: G01N 21/359, G01N 21/3563, G01N 21/25, G01N 21/85, G01N 21/27, G01N 21/47, G01N 33/10, G01J 3/02, G01N 21/84, G01N 21/03

(54) **GRAIN-COMPONENT SENSOR AND GRAIN-COMPONENT ANALYZING INSTRUMENT**

(30) Priority: 18.03.2022 JP 2022044313
(71) Applicant: Topcon Corporation, Tokyo 174-8580 (JP)
(72) Inventor: YAMAUCHI, Shun, Tokyo-to, 174-8580 (JP); AKIYAMA, Shugo, Tokyo-to, 174-8580 (JP)
(74) Representative: Louis Pöhlau Lohrentz

(57) **Abstract**

A grain-component sensor (3) comprises a white LED (17) for emitting a white light as a light emission source, a sample holder (8) in which a sample (9) is filled, a spectroscope (11) for receiving a reflected light from the sample and performing a spectroscopic analysis, and an optical filter (21) provided on an optical path between the white LED and the sample holder, the optical filter has an optical characteristic that is a transmittance of substantially 100% in a wavelength band from 950 nm to 1100 nm and cuts a light in a visible light region to substantially 0%, a light emitted from the white LED transmits through the optical filter and is irradiated to the sample as a detection light in a wavelength band from 950 nm to 1100 nm, and a diffused reflected light from the sample is condensed by a condenser lens (24) and is entered the spectroscope.

## Description

### BACKGROUND OF THE INVENTION

The present disclosure relates to a grain-component sensor and a grain-component analyzing instrument which analyzes components contained in grain by a spectroscopic method.

With grain as an object, an analyzing instrument which optically analyzes components contained in grain is known.

In recent agricultural machine such as a combine harvester, for instance, there is such a machine which has a GNSS sensor mounted and is capable of obtaining a positional information of a heavy machine during working.

A grain-component analyzing instrument is mounted on an agricultural machine, and by acquiring a quality data such as protein/moisture content rates in grain during harvesting in real time and by associating an acquired data with a positional information of a field, it is possible to conduct an adjustment of an application rate of a fertilizer or an improvement of a soil and to be utilized for efficient farming programs for producing crops with high and constant qualities.

Therefore, it is useful to mount the grain-component analyzing instrument on the agricultural machine for a development of an agriculture.

Conventionally, as a grain-component analyzing instrument, there is a grain-component analyzing instrument which irradiates grain with a near-infrared light, detects light absorption amounts in wavelength bands specific to water molecule, and to molecule constituting protein, and measures a moisture content rate and a protein content rate.

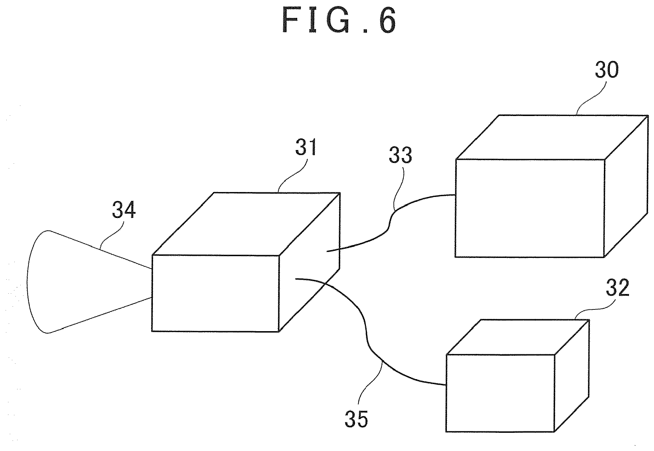

As shown in FIG.6, a conventional grain-component analyzing instrument includes a light source module 30, a measurement optical system 31 and a spectroscope 32, and a halogen lamp is used as a light emission source.

A light emitted by the light source module 30 (halogen lamp) is led to the measurement optical system 31 by an optical fiber 33 and is irradiated to an object to be measured (not shown) as a measuring light 34 from the measurement optical system 31. The measuring light 34 reflected by the object (reflected distance measuring light 34') is received in the measurement optical system 31 and is caused to enter the spectroscope 32 through an optical fiber 35. In the spectroscope 32, the reflected distance measuring light 34' is optically analyzed, a light absorption amount in specific wavelength bands are detected, and protein / moisture content rates of the grain are measured.

Since the halogen lamp used in the grain-component analyzing instrument as described above has a high heat generation amount and a high power consumption, the grain-component analyzing instrument needs a high heat shut-off structure and a heat radiation structure such that heat generation does not influence a measurement and thus, the light source module 30 and the measurement optical system 31, the spectroscope 32 have separate and independent constitutions, and each unit is connected by the optical fibers 33, 35. Therefore, there has been a problem that a size reduction of a grain-component analyzing instrument is difficult.

Further, a halogen lamp is expensive and has a short life, and there is also a problem that the halogen lamp should be frequently replaced, which has caused rises of maintenance fees and running costs.

### SUMMARY OF THE INVENTION

It is an object of the present invention to provide a grain-component sensor and a grain-component analyzing instrument which has a small size and is capable of reducing maintenance fees and running costs.

To attain the object as described above, a grain-component sensor according to the present embodiment comprises a white LED for emitting a white light as a light emission source, a sample holder in which a sample is filled, a spectroscope for receiving a reflected light from the sample and performing a spectroscopic analysis, and an optical filter provided on an optical path between the white LED and the sample holder, the optical filter has an optical characteristic that is a transmittance of substantially 100% in a wavelength band from 950 nm to 1100 nm and cuts a light in a visible light region to substantially 0%, a light emitted from the white LED transmits through the optical filter and is irradiated to the sample as a detection light in a wavelength band from 950 nm to 1100 nm, a diffused reflected light from the sample is condensed by a condenser lens and is entered the spectroscope.

Further, in the grain-component sensor according to a preferred embodiment, a light absorbing plate is provided on a reflection optical axis of the optical filter and absorbs a light reflected by the optical filter.

Further, the grain-component sensor according to a preferred embodiment further comprises a calibration module, the calibration module includes a standard white reflective board and a white-board insertion/removal module, and the white-board insertion/removal module is configured to be capable of inserting/removing the standard white reflective board into/from an optical path between the optical filter and the sample holder.

Further, in the grain-component sensor according to a preferred embodiment, the light emission source, the spectroscope, the optical filter and the calibration module are accommodated in a sensor case, and the sample holder can be detachably attached to the sensor case.

Furthermore, the grain-component analyzing instrument according to the present invention comprises any one of the grain-component sensors described above, a control module, a storage module, a display module and a power supply module, the power supply module supplies powers to the grain-component sensor, the control module, the storage module and the display module, the control module is configured to perform a spectroscopic measurement of the grain-component sensor based on a program stored in the storage module, to acquire a reflection diffusion spectrum based on an acquired spectroscopy data, and to display the reflection diffusion spectrum, a spectroscopy data and a calculation result on the display module.

According to the present embodiment, the grain-component sensor comprises a white LED for emitting a white light as a light emission source, a sample holder in which a sample is filled, a spectroscope for receiving a reflected light from the sample and performing a spectroscopic analysis, and an optical filter provided on an optical path between the white LED and the sample holder, the optical filter has an optical characteristic that is a transmittance of substantially 100% in a wavelength band from 950 nm to 1100 nm and cuts a light in a visible light region to substantially 0%, a light emitted from the white LED transmits through the optical filter and is irradiated to the sample as a detection light in a wavelength band from 950 nm to 1100 nm, a diffused reflected light from the sample is condensed by a condenser lens and is entered the spectroscope. As a result, it is possible to promote a size reduction of a light emission source and further, a size reduction, a power-saving, a longer life of a grain-component analyzing instrument, and maintenance fees and running costs are reduced.

### BRIEF DESCRIPTION OF THE DRAWINGS

FIG.1 is a schematic configuration drawing of a grain-component analyzing instrument according to an embodiment of the present invention.
FIG.2 is a graph illustrating an optical characteristic (wavelength transmission characteristic) of an optical filter of the present embodiment.
FIG.3 is a graph showing a dimming action of the optical filter.
FIG.4 is a diagram illustrating a reflective spectroscopy data at a calibration and a reflective spectroscopy data at an actual measurement.
FIG.5 is a graph showing an analysis data of the present embodiment and an analysis data of a conventional type.
FIG.6 is a schematic diagram of a conventional grain-component analyzing instrument.

### DETAILED DESCRIPTION OF THE PREFERRED EMBODIMENT

A description will be given below on an embodiment of the present invention by referring to the attached drawings.

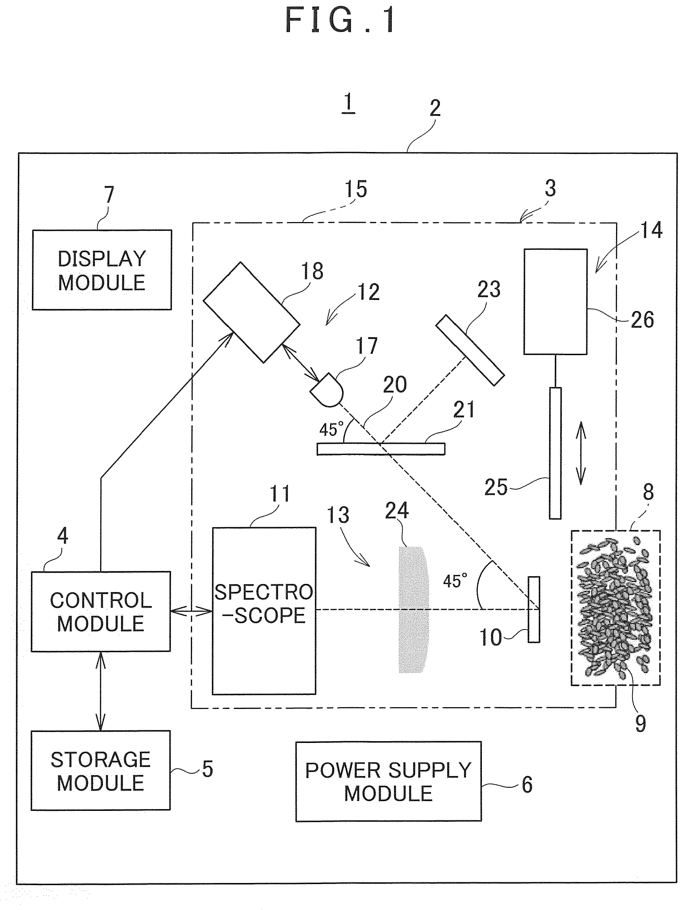

FIG. 1 shows a schematic configuration of a grain-component analyzing instrument 1 according to the present embodiment.

In FIG.1, a reference numeral 2 denotes a casing, and a component detection sensor 3, a control module 4, a storage module 5, a power supply module 6, a display module 7 and the like are provided inside the casing 2. The power supply module 6 supplies a power to the component detection sensor 3, the control module 4, the display module 7 and the like.

In the storage module 5, various types of programs for the grain-component analyzing instrument 1 to perform spectroscopic measurements and a program for the grain-component analyzing instrument to calculate content rates of a water, a protein and the like based on a spectroscopy data acquired by spectroscopic measurements are stored. Further, in the storage module 5, a spectroscopy data acquired by spectroscopic measurements, and a calculation result of content rates and the like are stored.

The control module 4 executes a program stored in the storage module 5 and executes required controls at required timings with respect to a spectroscope 11 (to be described later), a calibration module 14 (to be described later), a driver 18 (to be described later) and the like.

It is to be noted that, as the storage module 5, a semiconductor memory such as a RAM, a ROM, a FlashROM, a DRAM and the like, a magnetic recording memory such as a HDD and the like are used. Further, as the control module 4, a CPU specialized to the present embodiment or a general-purpose CPU, an embedded CPU, a microprocessor and the like are used.

A sample holder 8 is provided in the component detection sensor 3. The sample holder 8 may be provided fixedly with respect to the component detection sensor 3 or may be provided detachably.

It is configured such that an object to be measured (sample) 9 is filled in the sample holder 8, and when the sample holder 8 is provided fixedly to the component detection sensor 3, it is possible to fill the sample 9 in the sample holder 8 from an outside of the grain-component analyzing instrument 1 and it is possible to discharge from the sample holder 8.

Further, when the sample holder 8 is detachably attached to the component detection sensor 3, it is configured such that the sample holder 8 filled with the sample 9 is mounted on the component detection sensor 3 at a start of a measurement, and the sample holder 8 is removed from the component detection sensor 3 at a completion of a measurement.

Further, when a plurality of the sample holders 8 filled with the sample 9 are prepared in advance and the sample holder 8 is replaced at each completion of a measurement, a measurement can be performed efficiently.

The sample holder 8 has an incident window 10 into which a detection light is incident, and the incident window 10 is sealed by a transparent plate such as a glass plate and the like. Alternatively, the sample holder 8 may be entirely constituted by a transparent material.

Subsequently, a description will be given on the component detection sensor 3.

The component detection sensor 3 mainly includes the spectroscope 11, a light source module 12, a photodetector 13, the calibration module 14. The spectroscope 11, the light source module 12, the photodetector 13 and the calibration module 14 may be accommodated in a sensor case 15, and the component detection sensor 3 may be unitized. In this case, the sample holder 8 may be detachably attached to the sensor case 15 or may be detachably attached to the casing 2.

Electrical powers are supplied from the power supply module 6 to the spectroscope 11, the light source module 12, the photodetector 13 and the calibration module 14, respectively.

The spectroscope 11 has an energy-dispersive Si detector for which an Si single crystal is used as a detection element and is configured such that the detection element spectro-analyzes a received light. Further, a range capable of analyzing by a Si detector is a near-infrared light with a wavelength of 1100 nm or less.

The light source module 12 has a white LED 17 which emits a white light as a light emission source and a driver 18 which makes the white LED 17 emit light. An electrical power is supplied to the driver 18 from the power supply module 6, and a light emission is controlled by the control module 4. It is to be noted that a case in which a light emission source and the driver 18 are integrally constituted is shown, but the light emission source and the driver 18 may have a separate constitution.

An optical filter 21 is disposed on an optical path (optical axis) of the white LED 17. As described later, the optical filter 21 has an optical characteristic which shuts off or dims a light in a visible light region and transmits a wavelength band required for a component detection.

For the white LED 17, it is possible to use a white LED in a market, a light emitting element and a lens are integrated, and a light beam from the light emission element is set to be a parallel light or to have a predetermined spread angle. Further, in the present embodiment, since it is possible to shorten a light projection distance to the sample 9, it is possible to directly irradiate the sample 9 with a light emitted from the white LED 17 without going through an optical system such as a lens. Therefore, in the present embodiment, it is possible to omit a light-projecting optical system.

Subsequently, in the present embodiment, a water molecule and molecules constituting a protein, as contained in the sample 9, are detected by a spectroscopic analysis.

A water shows a relatively strong absorption at 1940 nm, 1450 nm, 1190 nm, 970 nm, and 760 nm. Further, a protein shows a complicated spectrum in which absorptions by various functional groups are overlapped, but it is known to have a characteristic absorption band at 2180 nm, 2050 nm.

Therefore, it is preferable to use a long wavelength of 1000 nm or more for a near-infrared spectroscopy, but an expensive InGaAs detector should be used for this wavelength band. Thus, in the present embodiment, a near-infrared spectroscopy is performed by using a wavelength range of a wavelength 1100 nm or less, and it is possible to use a Si detector.

A white light is emitted from the white LED 17, and the white light contains substantially the entire region of wavelength bands. It is preferred to shut off the light other than wavelength band required for detecting component. Further, as a characteristic of a white LED, since output of longer wavelength region than infrared region (900 nm) is drastically smaller than output of visible region (1/10, for instance), a saturation of a spectral sensitivity in a visible light region should be avoided.

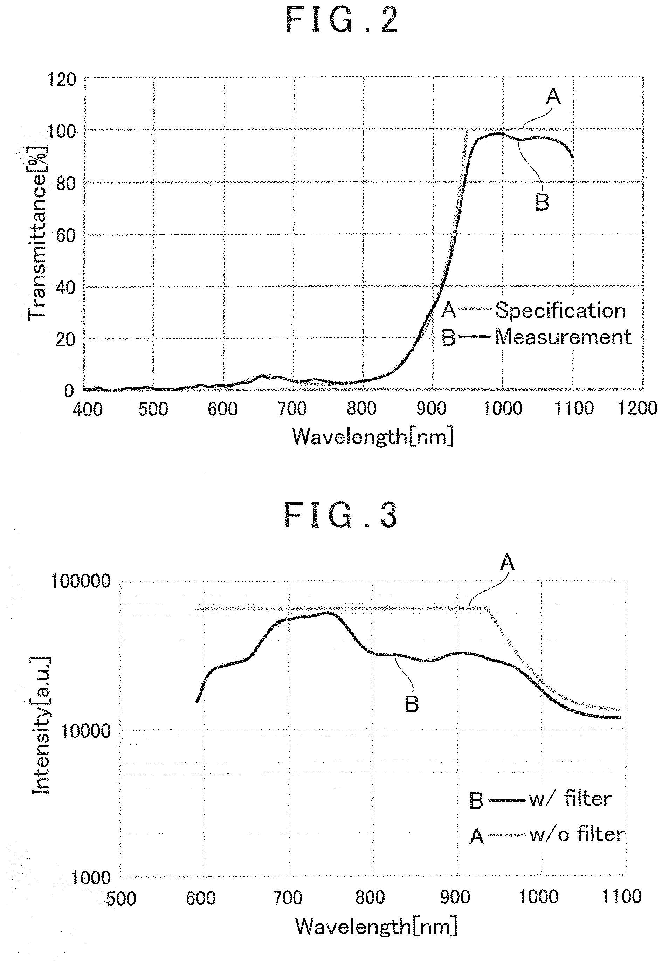

FIG.2 is a graph showing an optical characteristic of the optical filter 21, and a vertical axis indicates a transmittance and a horizontal axis indicates a wavelength.

Further, in FIG.2, a curve A is a
wavelength/transmittance curve required in the present embodiment, and a curve B is an actual
wavelength/transmittance curve of the optical filter 21 used in the present embodiment.

The curve A and the curve B substantially match, and the optical filter 21 has a transmittance of substantially 100% in a wavelength band from 950 nm to 1100 mm as used in the present embodiment.

That is, the optical filter 21 has an optical characteristic which transmits substantially 100% of a wavelength range of a wavelength from 1100 nm to 950 nm and cuts off a light in a wavelength region (visible light region) at 950 nm or less to approximately 0%.

In the present embodiment, an optical characteristic of the optical filter 21 is set to 2.5% transmittance with respect to a visible region and substantially 100% transmittance with respect to an infrared region. By allowing a transmission of 2.5% also with respect to a light in a visible region, as described later, it becomes possible to spectro-analyze molecules other than water molecules, protein molecules.

It is to be noted that, it is considered that an optimal transmittance of the optical filter 21 is different depending on the white LED 17 to be used and is not limited to a transmittance described above.

Therefore, a light irradiated the sample 9 has a wavelength range of a wavelength from 1100 nm to 950 nm. Since the irradiated light is limited to a wavelength band required for a component detection, an S/N of a detection result is improved. Further, it is also possible to avoid a saturation of a spectral sensitivity in a visible light region.

A light 20 emitted from the white LED 17 is transmitted through the optical filter 21 and is irradiated to the sample 9 through the incident window 10.

The optical filter 21 and the incident window 10 are inclined by a predetermined angle with respect to an optical axis of the white LED 17. Thereby, a light reflected by the optical filter 21 and the incident window 10 do not enter the white LED 17, and a light emission state in the white LED 17 is prevented from becoming unstable. It is to be noted that, in an illustration, the optical filter 21 and the incident window 10 are inclined by 45° with respect to an optical axis, respectively, but the inclined angle is not limited to this angle.

The optical filter 21 transmits a wavelength of 1100 nm or less, but at the same time, reflects a white light at a surface of the optical filter 21. In the present embodiment, a light absorbing plate 23 is provided on a reflection optical axis of the white light. The light absorbing plate 23 absorbs a reflected light from the optical filter 21. The light absorbing plate 23 prevents a light reflected by the optical filter 21 from irregularity reflecting inside the casing 2 and prevents a detection result by the spectroscope 11 from being influenced thereby.

A diffused reflected light reflected by the sample 9 and transmitted through the incident window 10 is condensed by a condenser lens 24 and is incident to the spectroscope 11.

The spectroscope 11 spectro-analyzes a reflected light being incident and outputs a spectroscopy data to the control module 4.

The control module 4 acquires a reflected diffused spectrum based on a spectroscopy data, further calculates contents of a moisture and a protein and displays calculation results of a reflected diffused spectrum, a spectroscopy data, a protein content rate, a moisture content rate, and the like on the display module 7.

Next, in the present embodiment, in order to guarantee a measurement accuracy, the calibration module 14 is provided.

The calibration module 14 has at least a standard white reflective board 25 and a white-board insertion/removal module 26.

The white-board insertion/removal module 26 inserts/removes the standard white reflective board 25 into/from an optical path of the light emitted from the white LED 17. Before and after a component analysis of the sample 9 or either one of before and after, or every predetermined time, the standard white reflective board 25 is inserted into an optical path between the optical filter 21 and the sample holder 8, the light 20 is irradiated on the standard white reflective board 25, and a reflected light from the standard white reflective board 25 is detected by the spectroscope 11 as a reference light for a calibration. Further, a driving of the white-board insertion/removal module 26 and a driving timing are controlled by the control module 4.

Next, a description will be given on a relationship between the optical filter 21 and the standard white reflective board 25.

As described above, in the present embodiment, a light beam in a wavelength region from 950 nm to 1100 nm (particularly, from 1000 nm to 1100 nm) is required, and when the white LED 17 is caused to emit a light such that a light intensity with a wavelength of this band is suitable for a measurement, a light intensity with a wavelength in a visible region (a wavelength at 950 nm or less) becomes strong, and a photodetector of the spectroscope 11 is saturated.

FIG.3 shows a reflected spectroscopy data of the standard white reflective board 25 acquired by inserting the standard white reflective board 25 into an optical path. In FIG.3, a vertical axis indicates an intensity of a light receiving signal, and a horizontal axis indicates a wavelength. Further, in the drawing, a curve A is a reflective spectroscopy data in a state without the optical filter 21, and a photodetector is saturated at a wavelength of 950 nm or less.

Next, a curve B shows a reflective spectroscopy data of the standard white reflective board 25 when the light 20 is emitted through the optical filter 21. The curve B shows that, by providing the optical filter 21, it is possible to acquire a reflective spectroscopy data without a saturation in all wavelength regions.

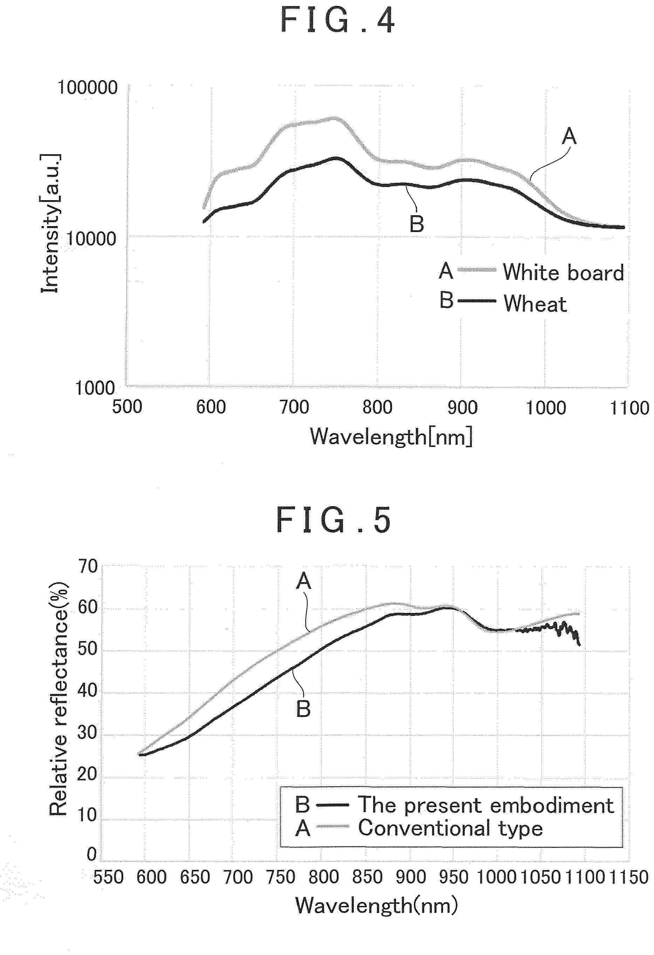

FIG.4 shows a reflective spectroscopy data of a standard white reflective board as a curve A and a reflective spectroscopy data from the sample 9 (wheat in the present embodiment) as a curve B, in a state where the optical filter 21 is provided. In FIG.4, a vertical axis indicates an intensity of a light receiving signal, and a horizontal axis indicates a wavelength.

A curve A shows a reflective spectroscopy data in a calibration state.

A curve B shows that it is possible to also acquire a light intensity required for a measurement with respect to a reflected light from a sample in all wavelength regions, and an effectiveness of the optical filter 21 is shown. Therefore, by a combination of the white LED 17 and the optical filter 21, it is possible to also perform a component analysis with respect to an object to be measured other than grain.

By performing a calibration, it is possible to detect a light emission state of the light source module 12 and abnormality of a measurement state, and the like.

FIG.5 shows measurement results of a grain-component analyzing instrument of a conventional type using a halogen lamp and of a grain-component analyzing instrument according to the present embodiment. In FIG.5, a vertical axis indicates a relative reflectance, and a horizontal axis indicates a wavelength. Here, a relative reflectance means that how much a light energy radiated from a colorimeter is reflected and returned from a test surface with respect to reflection from a reference surface is expressed by %.

With wheat as an object to be measured, a curve A is an analysis data of a grain-component analyzing instrument of a conventional type, and a curve B shows an analysis data of a grain-component analyzing instrument in the present embodiment.

Since measurement timings and samples are different, a simple comparison is difficult, but shapes of curves substantially match, and it is known that a measurement by a grain-component analyzing instrument in the present embodiment is useful.

As described above, a white LED 17 is used as a light source module in the present embodiment, and the white LED 17 as used is small with an outer shape of approximately 5 × 5 × 5.5 (mm), and further, a power consumption is 2.3 W. Further, a life is approximately 40000 Hr.

It is to be noted that a power consumption of a halogen lamp is 100 W, and further, a size of a power supply module by considering a heat radiation of a halogen lamp is approximately ϕ50 × 45 mm, and a life of a halogen lamp is approximately 1000 Hr.

Therefore, in the present embodiment, a light emission source itself is small, a power consumption is 2.3 W, a heat generation amount is drastically small, and a special heat radiation structure is not needed and thus, it is possible to markedly reduce a size of a light source module. Further, a spectroscope 11, a control module 4, a storage module 5, the display module 7 do not have to be separated but can be integrated and thus, it is possible to reduce a size and a weight of a grain-component analyzing instrument 1.

Further, since a life of the white LED 17 is markedly longer than a halogen lamp, it is possible to reduce maintenance fees and running costs.

In the present embodiment, it is possible to reduce sizes and weights of a grain-component sensor and a grain-component analyzing instrument and to realize an integration of a grain-component sensor and a grain-component analyzing instrument, and a power consumption is small and thus, it is easy to be mounted on a movable machine such as an agricultural machine.

## Claims

1. A grain-component sensor comprising: a white LED (17) for emitting a white light as a light emission source, a sample holder (8) in which a sample (9) is filled, a spectroscope (11) for receiving a reflected light from said sample and performing a spectroscopic analysis, and an optical filter (21) provided on an optical path between said white LED and said sample holder, wherein said optical filter has an optical characteristic that is a transmittance of substantially 100% in a wavelength band from 950 nm to 1100 nm and cuts a light in a visible light region to substantially 0%, a light emitted from said white LED transmits through said optical filter and is irradiated to said sample as a detection light in a wavelength band from 950 nm to 1100 nm, a diffused reflected light from said sample is condensed by a condenser lens (24) and is entered said spectroscope.

2. The grain-component sensor according to claim 1, wherein a light absorbing plate (23) is provided on a reflection optical axis of said optical filter (21) and absorbs a light reflected by said optical filter.

3. The grain-component sensor according to claim 1 or 2, further comprising a calibration module (14), wherein said calibration module includes a standard white reflective board (25) and a white-board insertion/removal module (26), and said white-board insertion/removal module is configured to be capable of inserting/removing said standard white reflective board into/from an optical path between said optical filter (21) and said sample holder (8).

4. The grain-component sensor according to claim 3, wherein said light emission source, said spectroscope (11), said optical filter (21) and said calibration module (14) are accommodated in a sensor case (15), and said sample holder (8) can be detachably attached to said sensor case.

5. The grain-component analyzing instrument, comprising the grain-component sensor according to any one of claims 1 to 4, a control module (4), a storage module (5), a display module (7) and a power supply module (6), wherein said power supply module supplies powers to said grain-component sensor, said control module, said storage module and said display module, wherein said control module is configured to perform a spectroscopic measurement of said grain-component sensor based on a program stored in said storage module, to acquire a reflection diffusion spectrum based on an acquired spectroscopy data, and to display the reflection diffusion spectrum, a spectroscopy data and a calculation result on said display module.
